# EUROPEAN PATENT SPECIFICATION

(11) **EP 3 912 665 B1**
(45) Date of publication and mention of the grant of the patent: **12.06.2024**
(21) Application number: 21167112.8
(22) Date of filing: 07.04.2021
(51) Int. Cl.: A61M 16/00, A61M 16/10, A61M 16/06, A61M 16/12, A61M 16/20, A61M 16/22

(54) **NON-INVASIVE VENTILATION DEVICE AND RECIRCULATION ASSEMBLY**
NICHT-INVASIVE BEATMUNGSVORRICHTUNG UND REZIRKULATIONSANORDNUNG
DISPOSITIF DE VENTILATION NON INVASIF ET ENSEMBLE DE RECIRCULATION

(30) Priority: 09.04.2020 IT 202000007660; 16.11.2020 IT 202000027396; 27.01.2021 IT 202100001598
(43) Date of publication of application: 24.11.2021
(73) Proprietor: Flow Meter S.p.a., 24040 Levate (Bergamo) (IT)
(72) Inventor: BELLANI, Giacomo, 20841 Carate Brianza (IT); COPPADORO, Andrea, 23900 Lecco (IT); PARATICO, Roberto, 24040 Levate (IT)
(74) Representative: Botti & Ferrari S.p.A.

(56) References cited:
- EP-A1- 1 852 137
- WO-A1-2013/096495
- US-A- 6 131 571
- US-A1- 2018 093 063
- US-B2- 10 420 909

## Description

### Field of Application

The object of the present invention is a non-invasive ventilation device as well as a recirculation assembly.

The present invention has particular application in the medical field and especially in the production of medical equipment in the ventilation field, preferably in the non-invasive ventilation field.

### Prior art

Non-invasive ventilation is a therapeutic treatment wherein, using specific devices connected to masks or hoods, the patient receives ventilation aid to the upper airways. Examples of non-invasive ventilation devices are disclosed in documents US 6 131 571, WO 2013/096495, US 2018/093063 and US 10 420 909.

An inexpensive and simple mode to provide an effective non-invasive ventilation aid is the so-called free-flow ventilation. In this mode, a flow generator transfers the medical gas (e.g. oxygen) coming from a source to a specific interface (mask or hood), to allow the patient to breath a controlled mixture having a predetermined concentration of medical gas; the inhaled gas is then released in the outside, directly or through a specific expiratory valve which puts in positive pressure the whole interface and therefore the upper airways of the patient.

Said type of ventilation is thus very advantageous due to its low cost, since it does not require sophisticated and expensive devices such as intensive therapy ventilators and, at the same time, it can be used in non-intensive care environments (hospital wards, ambulances).

Specifically, the CPAP apparatuses provide a flow of gas under pressure through a facial (or nasal) mask or a hood and a tube; the pressure of air possibly enriched with oxygen allows to improve the clinical conditions of the patient.

Said apparatuses generally comprise a "blower", i.e. a compression device which increases the air pressure of some cm of water, an element of connection to the patient and a tube which connects the "blower" to the latter.

CPAP apparatuses (Continuous Positive Airway Pressure) provide a constant positive pressure compared to the atmospheric pressure. These apparatuses also comprise apparatuses suitable for being employed on newborns using multiple interfaces which completely or partially adhere to the airways.

In addition to the devices that generate a continuous pressure (CPAP), more advanced apparatuses are available, which provide an additional pressure to the patient, such as V-PAP or B-PAP (Variable/Bilevel Positive Airway Pressure) and A-PAP (Automatic Positive Airway Pressure) apparatuses.

CPAP apparatuses are used in different fields; as home therapy for sleep apnea disorders, but also in hospital or pre-hospital settings for pathologies such as ARDS (Acute Respiratory Distress Syndrome), pneumonia, acute respiratory failure, post-operative hypoxemia, some cases of relapse of COPD (Chronic Obstructive Pulmonary Disease), the treatment of chest trauma with pulmonary contusions, pulmonary edema and atelectases of various origin. Moreover, they can be used as a prevention of re-intubation in patients who have just had removed a mechanical ventilator.

Acute respiratory failure can have various causes. When there is a damage at the lung parenchyma level, for example a damage associated to intra-alveolar exudation, interstitial edema, surfactant reduction or deficiency, the alveoli are more difficult to expand in the inspiratory step and are easier to collapse in the expiratory step. Alveolar distension can be partially supported with a pressure gradient (CPAP), between the respiratory tree and the outside, active for the whole duration of the respiratory cycle.

The pressure gradient prevents alveolar collapse at the end of the expiration, maintaining a higher volume at the end of the expiration and reducing the inspiratory effort, which results in an improvement in hypoxemia.

The use of a continuous flow of oxygen-enriched gas underlies not only the functioning of CPAP systems, but also the so-called High Flow Nasal Cannula (HFNC) therapy.

The interface which provides high-flow oxygen therapy consists of specific nasal cannulae which are fed with a gas mixture enriched with oxygen in a varying concentration (even up to 100%) with an adjustable flow, generally between 30 and 60 L/min.

In recent years, high-flow oxygen therapy has rapidly diffused due to its good tolerability by the patients, to its ease of use and to the effectiveness in treating several pathologies such as pneumonia, heart failure, post-extubation hypoxia.

Both CPAP systems and systems for high-flow oxygen therapy need to be fed with a high quantity of oxygen-enriched gas.

Different systems are available to generate an effective flow, such as for example Venturi systems, turbine systems and apparatuses which require compressed medical gases. Venturi systems are very widespread due to their low cost and ease of use.

However, the flow generators based on Venturi devices employed nowadays, even if they are advantageous, are difficult to handle as regards titration of oxygen (02) and the measurement of the gas flow delivered to the patient.

The devices and methods employed nowadays, even if advantageous, are still not precise enough as regards the titration of oxygen (02) and the dosage of air mixture to the patient.

Moreover, the dispersion into the environment of the gas used for free-flow ventilation results in both a high gas consumption and, in the case of possible harmful/infectious agents, in a dispersion of the latter in the environment, which, in particular medical health emergency or in the treatment of highly contagious pathologies, could lead to critical issues from both the purely medical and the managing standpoint.

The technical problem underlying the present invention is therefore to provide a non-invasive ventilation device and a recirculation assembly for said device which are able to get around the drawbacks of the above-mentioned prior art.

In particular, a purpose of the present invention is to provide a non-invasive ventilation device which optimizes the consumption of medical gas and is safer for the operators.

Moreover, a purpose of the present invention is to provide a recirculation assembly for a non-invasive ventilation device which is inexpensive and easily adaptable to devices of the prior art.

### Summary of the Invention

The invention is defined by the appended claims. Subject-matter referred as embodiments and/or disclosures, which are not covered by the claims are not part of the invention.

### Summary of the disclosure

The solution idea underlying the present disclosure is to provide an apparatus able to efficiently provide both the flow of a respiratory mixture and oxygen titration, to ensure an inhaled fraction FiO2 appropriate for the therapy underway.

The above-mentioned technical problem is solved by a device for non-invasive ventilation and/or for helping the patient to breath, comprising a flow generator provided with at least one supply port which can be connected to a source of a medical gas, and with at least one dispensing port and one patient interface element shaped to delimit a ventilation chamber which contains at least the patient's nose. Preferably, however, the interface element is shaped to delimit a ventilation chamber which contains both the nose and the mouth of the patient (mask) or the whole head (hood).

The interface element is provided with at least one inspiratory duct, in fluid connection with the flow generator, and one expiratory duct.

The device further comprises a recirculation circuit developing between said expiratory duct of the patient interface element and the flow generator.

The recirculation circuit is configured to recover at least a portion of said medical gas from an exhaled mixture (or expiratory flow) exiting from said expiratory duct.

Thus, the ventilation device defines a substantially closed circuit wherein the gaseous portion exiting from the ventilation chamber is recovered in its useful portion to be reintroduced upstream of the ventilation chamber.

Moreover, the recirculation circuit comprises a carbon dioxide absorber placed operationally upstream of the recovery port (i.e. of the flow generator) and configured to purify the exhaled mixture to be recirculated in the flow generator.

In addition, the pipe of the recirculation circuit comprises a vent placed operationally upstream of the carbon dioxide absorber and configured to avoid overpressure and negative pressure within said pipe.

Thereby, the medical gas (whether previously inhaled or not) pushed out of the ventilation chamber can be advantageously recovered and reused, thus minimizing gas waste.

Moreover, the flow generator is preferably equipped with a recovery port connected to the recirculation circuit to receive said portion of the medical gas and direct it towards the dispensing port. In other words, the flow generator has two inlet ports, a main one which receives "fresh" medical gas from the source and an auxiliary one (the recovery port) which receives the gas recovered by the recirculation circuit, thereby making them meet into the sole dispensing port.

Thereby, it is advantageously possible to use also flow generators which are already known and are adapted for circulating a fluid mixture of medical gases towards the interface element.

The vent is configured to avoid overpressure or negative pressure inside the system depending on the direction imposed by the conditions of pressure between the inside and the outside of the recirculation circuit.

Moreover, the device preferably comprises a regulating valve at the supply port, so as to limit and/or set the gas flow rate required by the application.

Also preferably, the regulating valve on the supply port is a knob-type throttling element to adjust the air sucked from the environment and therefore the inhaled fraction FiO2.

Preferably, there is also a pressurization valve configured to keep the pressure level in the ventilation chamber above a predetermined threshold value.

Said pressurization valve preferably comprises an inlet fitting, placed in (fluid) connection with the expiratory duct of the patient interface element, and an outlet fitting which leads into the pipe, preferably upstream of the vent (if present).

According to a preferred embodiment, the device according to the invention further comprises at least one first flow meter, associated with the flow generator through at least one supply fitting. Preferably, the at least one first flow meter is a high-flow flow meter able to reach flow rates of at least 15 L/m.

According to a more preferred embodiment, the device according to the invention further comprises a second flow meter, able to adjust the titration value of the flow, comprising a flexible fitting associable with the flow generator.

Preferably, said second flow meter is a high-flow flow meter able to reach flow rates of at least 15 L/m.

In a preferred embodiment, the first flow meter is integrated with the flow generator and generates an entrainment flow that determines the mixing of air and oxygen.

Moreover, the device according to the disclosure preferably comprises at least one flow selection element adapted for adjusting the oxygen titration value of said flow if a titration greater than 70% and up to 100% is required.

Preferably, the device according to the disclosure further comprises an oximeter connected in line to the flow generator and adapted for measuring and monitoring the oxygen titration degree in the flow.

Preferably, the device according to the disclosure further comprises at least one antimicrobial filter adapted for protecting the patient, further adapted for reducing the noise of said device.

According to a preferred embodiment, the carbon dioxide absorber comprises soda lime.

Preferably, said soda lime provides a granular conformation with hemispherical-shaped granules.

Alternatively, the carbon dioxide absorber comprises a lithium-based cell.

Alternatively, the carbon dioxide absorber comprises a solid element in a polymer matrix.

Preferably, the carbon dioxide absorber is an element which can be regenerated with the application of a heated air flow.

In an alternative embodiment, the carbon dioxide absorber comprises a zeolite element, a solid amine element or equivalent element.

More preferably, the carbon dioxide absorber comprises a zeolite 3A, 5A, Y, 13X element.

Even more preferably, the carbon dioxide absorber comprises a zeolite 13X element, which has a better adsorbing ability for the application.

Alternatively, the carbon dioxide absorber comprises a battery comprising carbon nanotubes for the absorption of carbon dioxide during the charging step.

Preferably, the carbon dioxide absorber is a double or multiple cell system, to allow said device to work in continuous even in the replacement or regeneration step of a cell of the carbon dioxide absorber.

Preferably, the device according to the disclosure further comprises an isolation and/or condensate collection filter.

Also preferably, the device according to the disclosure further comprises an absorption exhaustion monitoring electronic device.

Note that, preferably, in accordance with an aspect of the disclosure the recirculation circuit may define an independent expiratory kit, configured to engage between the patient interface element (preferably the expiratory duct) and the flow generator.

The expiratory kit comprises a pipe developing between a first end and a second end and a pressurization valve, a vent and a carbon dioxide absorber arranged sequentially along the pipe, wherein said first and second end are respectively shaped to engage in (or connect with) the expiratory duct of the patient interface element and the flow generator.

These and other characteristics, together with the related advantages, will be better highlighted by the following exemplary, thus non-limiting, description of a preferred, thus non-exclusive, embodiment of a non-invasive ventilation device and of an expiratory kit, with reference to the attached figures provided by way of example only, and not limitation.

### Brief description of the drawings

In the drawings:
Figure 1 shows a schematic representation of a first embodiment of a non-invasive ventilation device according to the present invention;
Figure 2 shows a schematic representation of a second embodiment of a non-invasive ventilation device according to the present invention;
Figure 3 shows a schematic representation of a third embodiment of a non-invasive ventilation device according to the present invention;
Figures 4 and 5 show schematic representations of a recirculation assembly for a non-invasive ventilation device according to the present invention, in two different embodiments;
Figure 6 shows a schematic representation of an embodiment of a non-invasive ventilation device comprising a double flow meter according to the present invention;
Figure 7 shows a schematic representation of an embodiment of a non-invasive ventilation device comprising a single flow meter according to the present invention;
Figure 8 shows a schematic representation of an embodiment of a non-invasive ventilation device comprising a flow selection element;
Figure 9 shows a schematic representation of an embodiment of a non-invasive ventilation device comprising a structure integrated with single flow meter according to the present invention;
Figure 10 shows a schematic representation of an embodiment of a non-invasive ventilation device comprising a structure integrated with single flow meter and a recirculation circuit according to the present invention;
Figure 11 shows another schematic representation of an embodiment of a non-invasive ventilation device according to the present invention;
Figure 12 shows another schematic representation of an embodiment of a non-invasive ventilation device according to the present invention.

### Detailed description

With reference to the attached figures, "1" indicates a non-invasive ventilation device according to the present invention.

Said device 1 can be connected to a source "S" of medical gas which can be of various, from both the fluid and the pressure standpoint, kind.

The source "S" of medical gas may be, for example, a source of gas at high or low pressure, which may be directly connected to device 1 in portable mode (e.g. cylinder with trolley, oxygen concentrator) or in static mode, through a conduit supply system typical of hospital facilities.

Also the medical gas may be of different kind, depending on the type of therapy or application.

In the preferred embodiment, the medical gas is oxygen, which is dispensed by the source at a predetermined pressure, which is later reduced, by specific adjustment devices, to levels which are optimal for therapy.

The medical gas might be a mixture comprising other kind of gases, such as for example compressed air, nitric oxide or an anesthetic gas (e.g. sevoflurane, nitrous oxide).

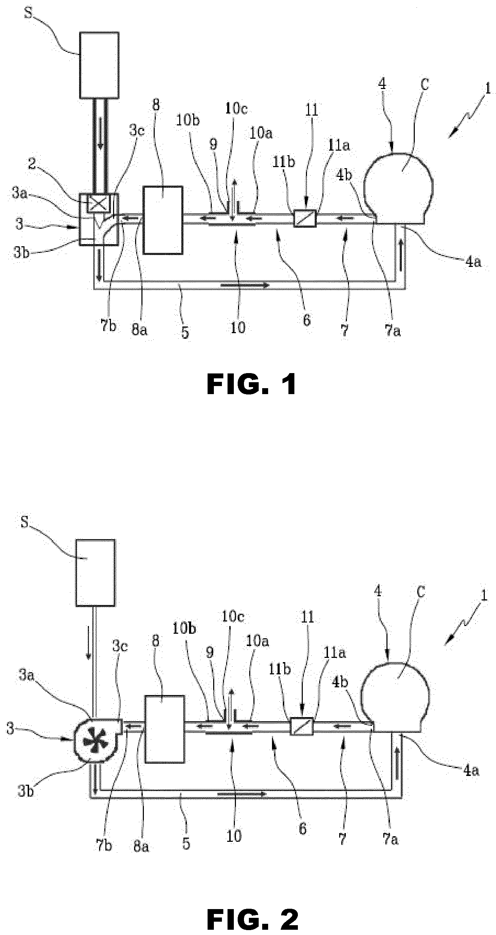

In the embodiment shown in Figure 1, for example, the device 1 comprises a regulating valve 2 which reduces the pressure of the medical gas coming from the source at high pressure.

Regardless of the embodiment, the device 1 comprises a flow generator 3 provided with at least one supply port 3a which can be connected to the source "S" of medical gas and with at least one dispensing port 3b.

The flow generator 3 is therefore a device configured to direct the medical gas coming to the supply port 3a towards the dispensing port 3b.

In the preferred embodiment, shown in Figure 1, the flow generator 3 is defined by a Venturi-effect-based system (e.g. a Venturi tube). Alternatively, it is also possible to use a turbine or a blower. Preferably, the regulating valve 2 is placed at the supply port 3a of the tube, so as to limit set the pressure to values which are appropriate to the gas flow rate required by the application/ therapy.

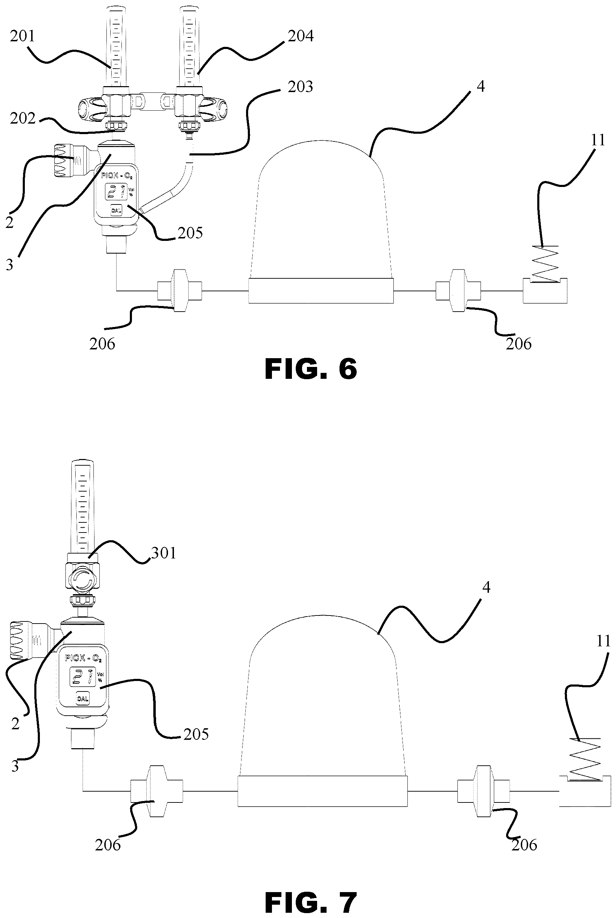

Therefore, said regulating valve 2, as can be seen in the exemplary embodiments in Figures 6 to 12, is an appropriate regulator adapted for adjusting a recirculation fraction, thereby allowing a correct shutting of the incoming air for dosing the mixture.

In other words, the regulating valve 2 on the supply port 3a is a throttling element, preferably but not limited to a knob-type one, which allows to adjust the air sucked from the environment and therefore the inhaled fraction FiO2.

Alternatively, as shown in Figure 2, the flow generator 3 might be defined by a turbine or, alternatively, by other manufacturing machines able to handle fluids in the form of a gas.

Moreover, in a preferred embodiment, which is shown in Figure 6, the flow generator 3 defined by the Venturi system, in the following for the sake of brevity also called Venturi device 3, is connected, by a supply fitting 201, to a first flow meter 202, directly interfaced to an oxygen source.

The first flow meter 202 is preferably a high-flow flow meter able to reach and manage flow rates of at least 15 L/min or even more.

Moreover, in the present embodiment, the Venturi device 3 is connected, by a flexible fitting 203, to a second flow meter 204. However, this solution is optional, as will be easy to understand from the rest of the description.

In the present embodiment, the supply fitting 202 and the flexible fitting 203 comprise each, respectively at the first flow meter 201 and at the second flow meter 204, an outlet fitting, for example of the type with thread 9/ 16" UNF (Unified Fine Threads). It is of course a non-limiting exemplary embodiment, since several alternative interconnection forms are possible.

The second flow meter 204 is a high-flow flow meter able to reach and manage flow rates of at least 15 L/min.

The first flow meter 201 acts as a "driver" source for the Venturi device.

The second flow meter 204 allows instead to adjust by increase the oxygen titration value of the mixture to be dispensed to the patient using the flexible fitting connected to the Venturi device.

Alternatively, an embodiment called embodiment with "single flow meter" is also possible, which is schematically shown in Figure 7, wherein the first flow meter 301 is a high-flow oxygen flow meter.

For example, a flow meter able to reach manage flow rates of at least 15 L/min and with double scale, i.e. 2-10 L/min and 6-50 L/min, may be employed.

The Venturi device 3 is again connected to the outlet of the first variable-area high-flow oxygen flow meter 301.

Also in this case, the first flow meter 301 acts as a "driver" source for the Venturi device.

In said embodiment, therefore, to obtain the correct oxygen titration there is a reduction of the sucked environment air instead of a further addition of oxygen.

Both the embodiments preferably have also an oximeter 205, connected in line to the Venturi device, arranged opposite the first flow meter 201, 301, which allows to constantly monitor the concentration of oxygen present in the mixture dispensed to the patient.

Moreover, an antimicrobial filter 206, in the exemplary embodiment shown two antimicrobial filters 206, is/are preferably also connected in line, to ensure protection of the patient and to allow to reduce the noise generated by the flow of the mixture, thereby also obtaining a better acoustic comfort for the patient during therapy.

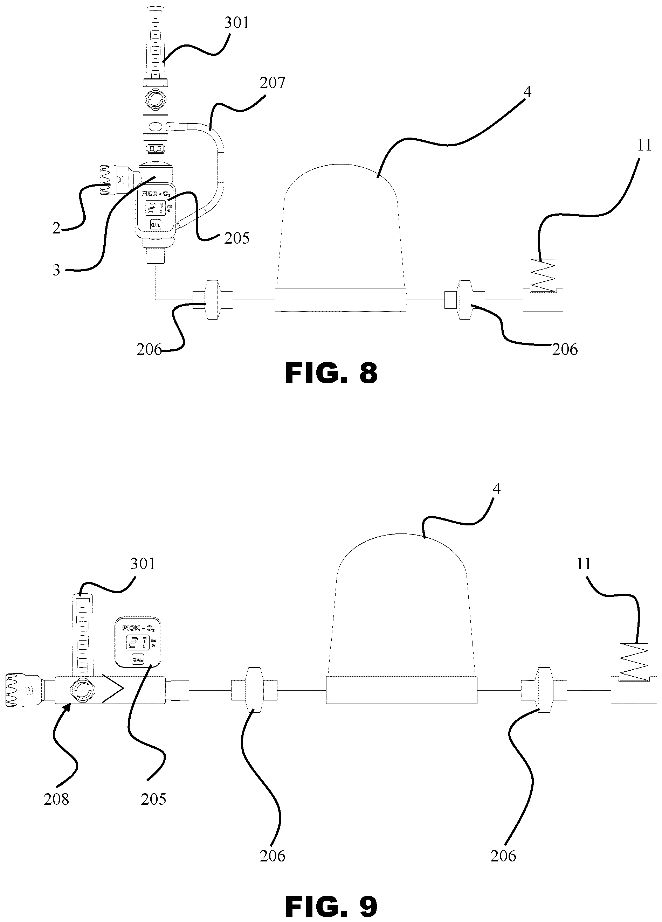

According to a further variant of the apparatus, shown in Figure 8, precarious clinical conditions, which require oxygen titration of the order of 70% or greater, and in particular up to 100%, may also be considered.

In said variant there is also a flow selection element 207 which allows to directly connect functionally the first flow meter 201, 301, with the patient, without passing through the Venturi device 3.

The flow selection element 207 is embodied by an alternative pipe which opens at the same time as the passage between the first flow meter 201, 301, and the Venturi device 3 closes, at the moment when a titration as above-mentioned is required.

Said solution is due to an intrinsic technical limitation in the use of the Venturi device 3 in the presence of a single flow meter.

In fact, if a FiO2 of the order of 70% or greater, and in particular up to 100%, is required, in the presence of a single flow meter, the total opening of the flow of the first flow meter 201, 301, and the closing of the regulating valve 2 as throttling element is not sufficient, since the injector inside the Venturi device 3 would in any case limit the inflow of pure oxygen. Therefore, the only approach for this extreme case is to bypass the Venturi device 3 by creating an alternative circuit.

This mode type, defined "flush", allows to feed the system with flow rates of even 70 L/min pure oxygen.

According to a third embodiment, shown in Figure 9, the present invention provides for an apparatus with an integrated structure 208 comprising a first flow meter 201, 301, which generates an entrainment flow that determines the mixing of air and oxygen; for example, it is possible to employ a high-flow oxygen flow meter to manage flow rates of at least 50 L/min and with double scale, i.e. 2-10 L/min and 6-50 L/min.

The first flow meter 201, 301, is further integrated with the Venturi device 3. It is therefore possible, with a single device, the use in CPAP therapy and the high-flow oxygen therapy (HFNC).

Also according to the present embodiment, the Venturi device works as a flow generator and amplifier and only requires feeding from the oxygen source S.

The integrated structure 208 provides also for a hooking clamp for a secure connection.

At the inlet to the integrated structure 208 there is preferably a connection with gas thread specific for the connection to the oxygen source S by a flexible tube (not shown), the latter being provided with an appropriate coupling to the distribution plant of the user and immovably fixed.

Also the present embodiment provides for an oximeter 205, also integrated in the integrated structure 208 and in line with the first flow meter, which allows to constantly monitor the concentration of oxygen in the mixture dispensed to the patient and, connected in line, an antimicrobial filter 206.

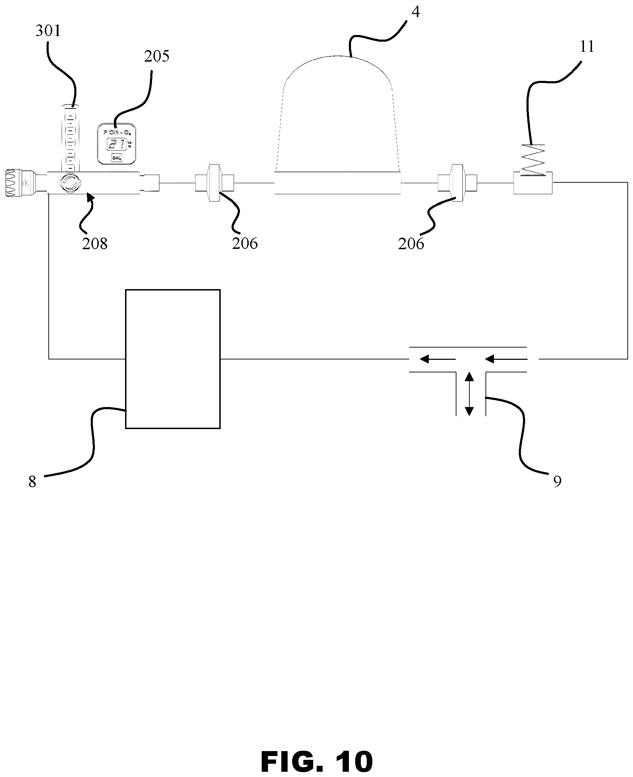

Figure 10 shows the whole apparatus, in which said integrated structure 208 has been inserted.

A patient interface element 4 is located downstream of the flow generator 3.

Preferably, between the flow generator 3 and the patient interface element 4, there is a conduit or channel 5 which transfers the medical gas towards the patient along a route adaptable to the hospitalization and/or use conditions.

In this regard, the conduit or channel is preferably flexible, even more preferably it is a corrugated tube.

The patient interface element 4 is shaped to delimit a ventilation chamber "C" which contains at least the patient's nose, so as to isolate the upper airways from the outside providing a predetermined gas mixture (e.g. high-concentration oxygen) which is useful to the therapy which is performed.

Preferably, the interface element 4 is shaped to delimit a ventilation chamber which contains both the nose and the mouth of the patient (mask) or, in some cases, the whole head (hood).

In particular, the patient interface element 4 is provided with at least one inspiratory duct (or port) 4a, in fluid connection with said flow generator 3, and one expiratory duct (or port) 4b.

The inspiratory duct 4a receives therefore the medical gas from the flow generator 3, whereas a flow of gaseous mixture exhaled by the patient passes through the expiratory duct 4b.

Note that, structurally, the two ducts (inspiratory 4a and expiratory 4b) might share a mouthpiece with the ventilation chamber "C", separating only in a distal zone (i.e. more external in relation to) from the ventilation chamber "C".

In the preferred embodiment, the patient interface element 4 is a nasal mask, an oronasal mask, nasal cannulae or a CPAP (Continuous Positive Airway Pressure) hood, whose concave conformation defines the ventilation chamber "C".

In said embodiments, the patient interface element 4 has preferably two distinct ducts, an inspiratory one 4a and an expiratory one 4b, so as to optimize the gaseous flow in the correct direction.

Of course, also according to future development of medical therapies, it could be possible to use different connection elements.

In an alternative embodiment (not shown), it is indeed possible to use a high-flow nasal cannula HFNC, with which it is possible to perform a high-flow oxygen therapy, thereby providing a non-invasive respiratory help with release of heated, humidified, and oxygen-enriched air to the patient.

This allows to improve oxygenation, the respiratory rate, dyspnea and the comfort of the patient and helps in accelerating rehabilitation of the patient after extubation.

According to an aspect of the invention, the device 1 comprises a recirculation circuit 6 developing between the expiratory duct 4b of the patient interface element 4 and the flow generator 3.

Said recirculation circuit 6 is configured to recover at least a portion of said medical gas from an expiratory flow (i.e. from the exhaled mixture) exiting from the expiratory duct 4b.

Advantageously, this allows to avoid (or limit) wasting medical gas, which, in specific emergency conditions or in case of scarcity of raw material, may be critical to the success of therapy.

Moreover, in the case of anesthetic medical gases or of infectious pathologies of the patient, minimizing the exhaled mixture (or expiratory flow) released into the environment is an important result also from the standpoint of safety and health of health workers.

In this regard, note that the flow generator 3 is preferably equipped with a recovery port 3c connected to the recirculation circuit 6 to receive said portion of the medical gas and direct it towards the dispensing port 3b.

In the preferred embodiments, the recovery port 3c is defined by an auxiliary channel which engages between the supply port 3a and the dispensing port 3b, preferably oriented so as to facilitate the outflow of the recovered gas towards the dispensing port 3b.

Preferably, the recirculation circuit 6 comprises a pipe 7 developing between a first end 7a and a second end 7b.

The first end 7a is connected to the expiratory duct 4b of the patient interface element 4.

The second end 7b is connected to the flow generator 3, preferably to the recovery port 3c.

Preferably, one or more devices adapted for optimizing the functionalities of the recirculation circuit 6 are arranged along the pipe 7.

In this regard, the recirculation circuit 6 preferably comprises a carbon dioxide absorber 8 arranged along the pipe 7.

More in particular, it is configured to purify the medical gas to be recirculated in the flow generator 3.

The carbon dioxide absorber 8 may be of different kind, but it preferably comprises an inlet port and an outlet port between which there is a filtering chamber 8a.

The filtering chamber 8a preferably comprises a rechargeable cartridge made of absorbent material, such as, for example, soda lime, lithium hydroxide, zeolites or the like.

Even more specifically, therefore, the carbon dioxide absorber 8 is adapted for purifying said gases exhaled by the patient.

However, it is possible to employ several absorber elements coupled to each other.

According to a preferred embodiment, the carbon dioxide absorber 8 comprises soda lime.

Soda lime consists of a mixture of calcium hydroxide (Ca(OH)2), sodium hydroxide or caustic soda (NaOH), water (H2O). Some compounds contain potassium hydroxide (KOH). The reaction which occurs is the following:

CO2 + H2O → H2CO3

H2CO3 + 2Na(OH) = Na2CO3 + H2O + heat

Na2CO3 + Ca(OH)2 = CaCO3 + 2Na(OH) + heat

The reaction with KOH instead of Na(OH) is the same. Water is necessary at the beginning of the reaction; the reaction produces 1 mole H2O for each mole of neutralized CO2.

Caustic soda is an intermediate which is regenerated: it is responsible for the alkaline pH of the initial mixture.

The intergranular space is the volume of the gas contained between the granules in the carbon dioxide absorber 8 after filling.

According to the type of granules, this space is from 15 to 45 % of the inner volume of the carbon dioxide absorber 8. Granules compression allows to reduce the space, thereby resulting in intragranular space and volume of gas in the granules of about 2-4 %.

Soda lime contains a colored indicator which is an acid or a base which changes color with a pH variation. The ideal indicator should change color at the beginning of the pH variation, should be nontoxic and stable.

The dye used is ethyl violet which changes color from white to violet.

Alternatively, it is possible to use potassium permanganate, which changes color from green to white.

The transformation of caustic soda into carbonate causes the color change of the colored indicator.

According to a preferred embodiment, soda lime has a higher percentage of sodium hydroxide, in particular higher than 1, and a higher density of product in a same absorber, in particular a density higher than 70 g/ml.

Therefore, the overall dimensions being equal, a higher absorption efficiency is obtained.

According to another preferred embodiment, soda lime provides a granular conformation with hemispherical-shaped granules.

The hemispherical shape allows for a low level of respiratory resistance, thereby extending periods of use.

Said shape further ensures a high ability of CO2 absorption.

Moreover, the hemispherical shape ensures a high scratch resistance, thereby reducing powder formation.

According to an alternative embodiment, the carbon dioxide absorber 8 comprises a lithium-based cell.

In particular, in this case calcined lithium hydroxide is used, which reacts with carbon dioxide at room temperature.

Specifically, it allows to absorb carbon dioxide 8 from steam according to the chemical reaction:

2LiOH + CO2 → Li2CO3 + H2O.

Calcined lithium hydroxide ensures reliable performance and provides for high ability of CO2 absorption, in fact, 1.5 Kg lithium hydroxide absorb about 1 Kg CO2.

According to an alternative embodiment, the carbon dioxide absorber 8 comprises a solid element in a polymer matrix.

By using a solid element, the drawbacks of channelization are so avoided.

Moreover, carbon monoxide emissions are reduced and problems of accidental caustic powder of granular-type absorbents are eliminated.

Absorbent powders are bound by a polymer matrix, thereby also allowing a rapider and easier cleaning of the system.

Said solution is suitable for all gas flow rates.

According to another alternative embodiment, the carbon dioxide absorber 8 comprises a battery comprising carbon nanotubes for the absorption of carbon dioxide 8 during the charging step.

In particular, the carbon dioxide absorber 8 uses reactive electro-oscillating adsorption in the solid state comprising an electrochemical cell which uses the reducing addition of CO2 to quinones for carbon capture.

An electrochemical cell with a negative composite electrode made of carbon nanotubes captures CO2 during charging by carboxylation of reduced quinones and releases CO2 when it is discharged. Preferably, the carbon dioxide absorber 8 is a double or triple cell system.

Preferably, the cells are radial-flow cells so as to reduce load losses.

Advantageously, moreover, the system according to the disclosure may also have a unit of automatic switch, which is able to recognize the saturation reached by a cell and to consequently redirect the flow of air to be filtered to a cell which is still not saturated.

Therefore, by using a double or multiple cell system, it is possible to ensure a continuous functioning of the system, since when one of the cells reaches saturation it is replaced, while the system continues to work passing through a cell which is still not saturated.

This enables a controlled increase of performance, continuity of the recirculation system during replacement or regeneration.

According to a preferred embodiment, the carbon dioxide absorber 8 is further an element which can be regenerated with the application of energy, in particular of a heated air flow.

In particular, a fan, associated to a heating resistance, which generate in functional coupling a heated air flow, is used. Said heated air flow is blown onto the battery towards the environment, thereby entraining CO2 which is released from the carbon dioxide absorber 8 as a result of heating. Thereby, purification and regeneration of the cell are obtained. The only parameters to control to obtain said regeneration are the temperature of the heated air flow and the regeneration time.

According to another alternative embodiment, in fact, the carbon dioxide absorber 8 comprises an element containing zeolites, solid amines or analogous chemical compounds able to both absorb carbon dioxide and to release it depending on the fact of being subjected to suitable stimuli, such as, indeed, the increase of temperature by using a warm air flow or by directly applying heat by thermal resistance.

Amines generally react with CO2 to form carbamate, according to the reaction:

CO2 + 2 RNH2 ↔ RNH3++ RNHCOO-

In the case of a double or multiple cell system, as above mentioned, the system according to the disclosure may also comprise the unit of automatic switch, and it is therefore possible to provide for the regeneration of the saturated cell while the system continues to work passing through a cell which is still not saturated and thereby to optimize functioning by avoiding forced inactivity times.

In an alternative embodiment, the carbon dioxide absorber comprises a zeolite 3A, 5A, Y, 13X element.

Even more preferably, the carbon dioxide absorber comprises a zeolite 13X element, which has a better adsorbing ability for the application.

This solution allows to obtain high autonomy, excellent efficiency, and of course a much lower ecological impact, in addition to a low cost.

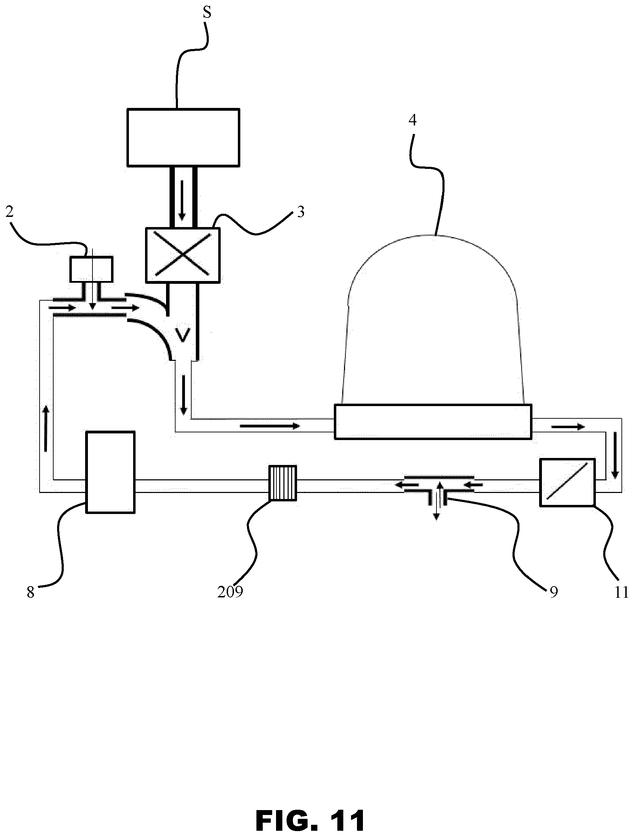

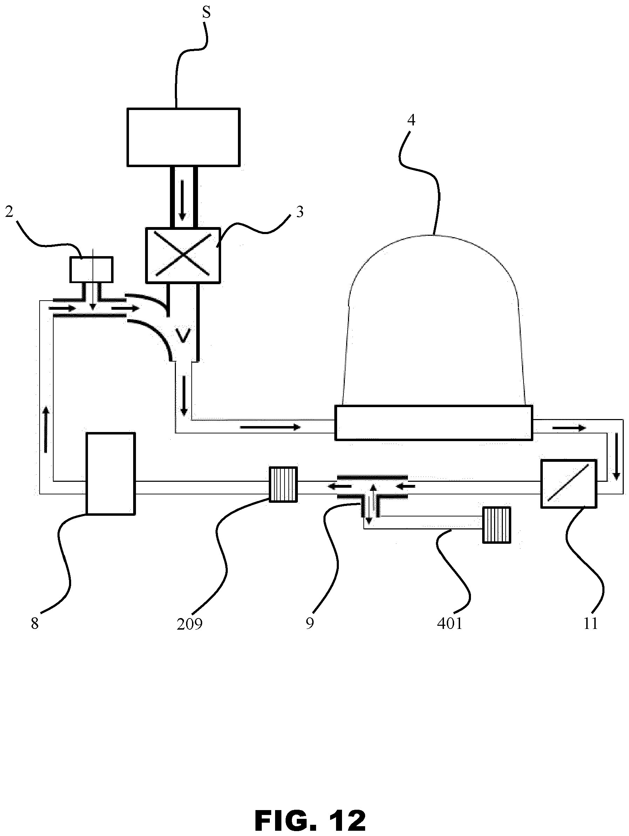

Moreover, as can be observed for example in Figures 11 and 12, the circuit preferably comprises an isolation and/or condensate collection filter 209.

It is therefore possible to collect biological material coming from the patient's airways in a non-invasive way.

It is known that the gas exhaled by a subject contains, in addition to steam, also various substances which may be indicative of pathological processes which are occurring, in particular processes in the lungs.

The analysis of the condensed exhaled gas was deemed to be promising for the diagnosis of pathogenic agents, neoplasia and other biological markers.

However, currently, the use of condensate of exhaled gas as a biological sample is still not common practice.

The sample of condensate of exhaled gas may be collected when the subject is treated in an invasive manner, or by exhalation in specific instruments.

Therefore, thanks to said isolation and/or condensate collection filter 209, it is possible to arrange a device for the collection of the exhaled gas in a position along the expiratory portion of the circuit, preferably upstream of the CO2 absorber.

Said device may be provided with specific supports able to keep back for subsequent analyses, i.e. to recognize in real time the presence of specific biological markers.

Advantageously, it is therefore possible to make a diagnosis in a non-invasive manner which is also well-tolerated by the patient.

Moreover, it is possible to provide diagnostic instruments which can be used very early during the course of treatment, possibly even in non-hospital setting.

According to an alternative embodiment, it is possible to use an electronic device for monitoring carbon dioxide concentration (not shown) to promptly identify the loss of efficiency of the CO2 absorber.

In fact, when using caustic soda there is sometimes the drawback that, after stopping the use, the colored indicator slowly loses color due to exchange of products of the reaction of the periphery with the inside of the lime granule. It may thereby seem that the lime is still active even though it is exhausted. Therefore, the color of the soda lime used is not a good indicator of its effectiveness.

Differently, by using the monitoring electronic device, the precision and certainty of real exhaustion are greater.

Moreover, it is also possible to provide an emergency unit which excludes recirculation in case anomalies in functioning are detected inside the system.

Preferably, as can be observed in detail in both Figures 1 and 2 and in Figures 10, 11 and 12, a vent 9, or relief valve, is provided, which is configured to avoid that overpressure or negative pressure are generated inside pipe 7.

Even more specifically, it is a suction and vent duct 9 upstream of said carbon dioxide absorber 8.

The suction and vent duct 9, or simply vent 9, is actually configured to avoid both negative pressure and overpressure inside the system.

In fact, in the case there is a negative pressure inside the recirculation circuit, and therefore an insufficient amount of mixture in the recirculation circuit, this may be mitigated by a suction directly from the outside through the suction and vent duct 9.

Therefore, said suction and vent duct 9 has a dual function depending on the direction imposed by the conditions of pressure between the inside and the outside of the recirculation circuit.

In other words, as can be observed looking the directional arrows representing air inlet and outlet in the Figures, if there is a higher pressure inside the system compared to outside conditions, a vent function is provided, whereas if there is a negative pressure inside the system compared to the outside conditions, a suction function is provided carrying out a sort of compensation inside the system.

Moreover, in an alternative embodiment shown in Figure 12, a protuberant element 401 associated to the suction and vent duct 9 is also provided.

Preferably, the protuberant element 401 is represented by a corrugated-tube element, more preferably by a double-tube element, even more preferably by a coaxial-double-tube element, which, in the above-mentioned vent function, fills with gas mixture, whereas, in the suction function, it discharges into the system said mixture with which it has filled. It is thereby provided a further optimization, since a gas mixture, and not simple environment air, is sucked into the circuit, said gas mixture having more positive effects on the patient.

Preferably, the suction and vent duct 9 is placed operationally upstream of the carbon dioxide absorber 8.

In the preferred embodiment, said suction and vent duct 9 is defined at least in part by a joint or fitting 10 having three end portions which are (directly or indirectly) connected to, respectively, the expiratory duct 4b, to the flow generator 3 (or to the absorber 8) and to the outside (or other discharge environment).

In particular, the joint or fitting 10 comprises:
- a first (inlet) end portion 10a, connected to the expiratory duct 4b to receive the exhaled mixture;
- a second (outlet) end portion 10b, connected to the flow generator 3 and/or to the carbon dioxide absorber 8 to deliver them the exhaled mixture;
- a third (discharge) end portion 10c, to release excess gas volume in the case of overpressure.

In the preferred embodiment, said third end portion 10c is provided with a filter configured to limit the release of harmful particles into the outside; preferably, the filter is of the HEPA (High Efficiency Particulate Air filter) type.

Preferably, the joint or fitting 10 is a T-joint or a Y-joint.

Preferably, as schematically illustrated in Figures 1 and 2, the suction and vent duct 9 may be connected to the patient interface element 4 and/or to the carbon dioxide absorber 8 by flexible conduits (for example corrugated conduits) which define in part pipe 7.

Thereby, both positioning the recirculation circuit 6 and its adaptation to preexisting ventilation systems become easier (as it will be better explained below).

Preferably, moreover, the recirculation circuit 6 comprises a pressurization valve 11 configured to keep the pressure level in the ventilation chamber "C" above a predetermined threshold value.

In particular, the pressurization valve 11 is preferably arranged along the pipe 7, operationally upstream of said suction and vent duct 9.

The pressurization valve 11 comprises an inlet fitting 11a and an outlet fitting 11b.

The inlet fitting 11a is placed in fluid connection with the expiratory duct 4b of the patient interface element 4.

The outlet fitting 11b is preferably placed in connection with the suction and vent duct 9.

Moreover, the pressurization valve 11 comprises an active (or passive) element configured to keep the pressure at a predetermined level corresponding to the threshold value.

In the preferred embodiments, the threshold value is between 0-50 cmH2O, more preferably between 5-15 cmH2O.

Preferably, the pressurization valve 11 is defined by a PEEP (Positive end expiratory pressure) valve provided with said inlet and outlet fittings.

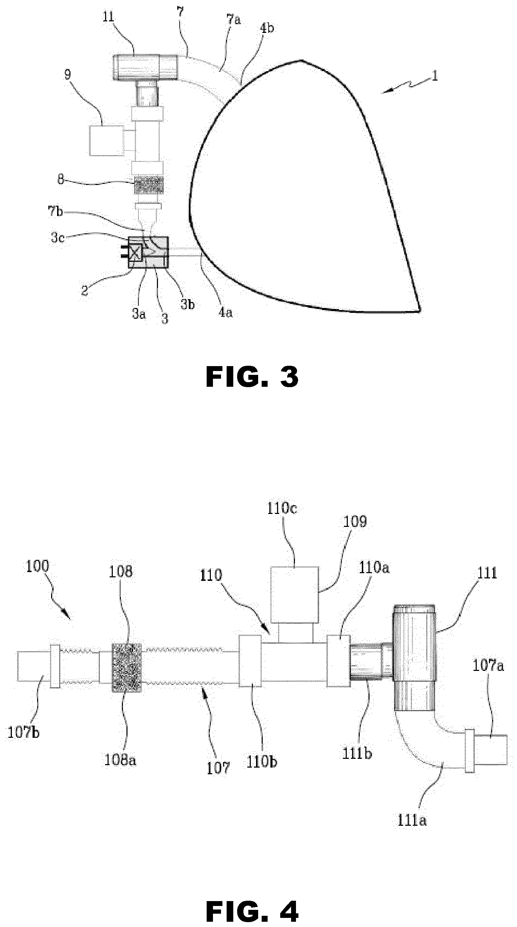

Note that, with reference to the embodiment of Figure 3, the device might have a very compact conformation, with substantially no connection conduits or channels between the flow generator 3 and the inspiratory duct 4a nor along the recirculation circuit 6.

In said embodiment, all the components are connected in a substantially rigid way to the patient interface element 4, so as to limit the overall dimensions and facilitate portability.

Another object of the present disclosure are also a recirculation assembly (which is also claimed) and kit 100 (not claimed) for a non-invasive ventilation device, preferably for but not limited to the device 1 described above.

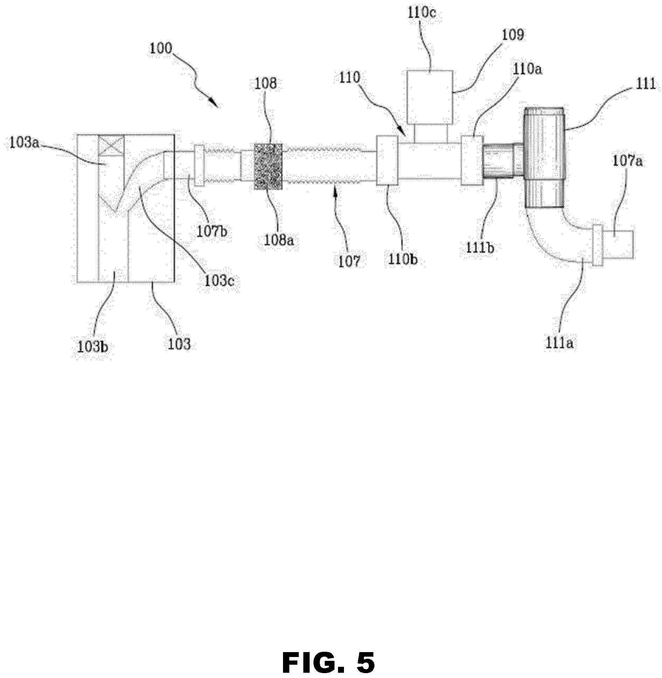

Hereinafter, the recirculation assembly 100 (shown in Figures 4 and 5) will be briefly described, pointing out now that, for all the characteristics shared with the previously described device 1, the same specifications and peculiarities will be applied mutatis mutandis.

Moreover, in order to explain more clearly, even if the components of the recirculation assembly 100 are defined with the same denomination as those of the recirculation circuit 6 of device 1, they will be identified with different, although thereto attributable, reference numerals.

In particular, the recirculation assembly 100 comprises a pipe 107 developing between a first end 107a and a second end 107b.

Said pipe 107 has preferably one or more flexible and/or extendable portions which allow its adaptation to preexisting ventilation systems.

Moreover, the first 107a and the second end 107b of the pipe 107 are shaped to engage in the expiratory duct 4a of the patient interface element 4 and in the flow generator 3 (in particular in the recovery port 3c), respectively.

In this regard, the first 107a and the second end 107b of pipe 107 are preferably provided with respective joints, which, in the preferred embodiment, are of the type compliant with regulation EN ISO 5356, preferably of 22 or 15 mm caliber.

The kit 100 further comprises a carbon dioxide absorber 108 arranged along said pipe 107, whose essential and/or optional characteristics are analogous to those of the carbon dioxide absorber 8 previously described.

A suction and vent duct 109 is placed operationally upstream of the carbon dioxide absorber 108, said suction and vent duct being configured to avoid overpressure or negative pressure within said pipe 107; also in this case, the essential and/or optional characteristics of the suction and vent duct are analogous to those of the suction and vent duct previously described.

In a position operationally between the first end 107a of the pipe 107 and the suction and vent duct 109 there is a pressurization valve 111 configured to keep the pressure level in the first end 107a above a predetermined threshold value.

Preferably, moreover, the recirculation assembly 100 further comprises a flow generator 103 provided with at least one supply port 103a which can be connected to the source S of a medical gas, with at least one dispensing port 103b and with one recovery port 103c connected to the second end 107b of the pipe 107.

Advantageously, it is thereby possible to replace the flow generator of the preexisting ventilation device with that of the recirculation assembly 100, specifically designed to allow the inflow of the recovered gas into the dispensing port 103b.

With reference to the recirculation kit, the components are the same that were already described in the recirculation assembly, but they are separate from each other and connectable only in the operative configuration.

The invention achieves the desired purposes and accomplishes important advantages.

In fact, the provision of a recirculation circuit associated to appropriate couplings and fittings in the inspiratory circuit allows to both optimize consumption of medical gas (e.g. oxygen) and to minimize dispersion of the exhaled mixture in the environment.

Moreover, the use of a pipe provided with a pressurization valve, with a vent and with a carbon dioxide absorber allows to provide an independent expiratory kit adaptable to any preexisting non-invasive ventilation system, thereby facilitating consumption optimization without the need of expensive investment by the owners of prior art systems, whether they are patients or healthcare premises.

Advantageously, the present system may be applied in closed-loop non-invasive ventilation systems or also systems for helping the patient to breath that remove excess carbon dioxide, such as for example ventilator circuits for invasive ventilation used in systems which are specific for dispensing anesthetic gas. For example, during a general anesthesia, it is common practice to use the closed-circuit or partially-closed-circuit mode, wherein a part of the gas exhaled by the patient is reused to reduce consumption of anesthetic gases. Usually, a container of soda lime is arranged along the circuit.

When the CO2 meter placed along the recirculation circuit reaches a preset level, said container has to be manually replaced to keep homeostasis of the patient, therefore the recirculation circuit needs to be stopped when the replacement occurs.

Advantageously, the use of the present disclosure in the above-described multiple-cell embodiment, also defined embodiment "with multiple absorption beds", allows, in the case one of the beds exhausts its ability of absorbing CO2, to keep functioning the recirculation of anesthetic gas by redirecting the recirculating gas to the non-exhausted bed. Moreover, as already said, said deviation may occur automatically, without the need of user's intervention.

Even more advantageously, the present disclosure in the embodiment "with multiple regenerable absorption beds" allows the user not to replace any container, since the exhausted one is automatically regenerated to become again ready-for-use.

## Claims

1. A device for non-invasive ventilation and/or for helping the patient breathing, comprising:
- a flow generator (3) provided with at least one supply port (3a) which can be connected to a source (S) of a medical gas and with at least one dispensing port (3b);
- a patient interface element (4) shaped to delimit a ventilation chamber (C) which contains at least the patient's nose and provided with at least one inspiratory duct (4a), in fluid connection with said flow generator (3), and one expiratory duct (4b);
- a recirculation circuit (6) developing between said expiratory duct (4b) of the patient interface element (4) and the flow generator (3) and configured to recover at least a portion of said medical gas from an exhaled mixture exiting from said expiratory duct,
wherein said recirculation circuit (6) comprises a pipe (7) developing between a first end (7a), connected to the expiratory duct (4b) of the patient interface element (4), and a second end (7b), connected to the flow generator (3); said recirculation circuit (6) further comprising a carbon dioxide absorber (8) arranged along said pipe (7), and
wherein the pipe (7) comprises a vent (9) placed operationally upstream of the carbon dioxide absorber (8) and configured to avoid both negative pressure and overpressure inside the recirculation circuit.

2. The device according to claim 1, wherein said flow generator (3) is equipped with a recovery port (3c) connected to the recirculation circuit (6) to receive said portion of the medical gas and direct it towards the dispensing port (3b).

3. The device according to claim 1 or 2, wherein said vent (9) is configured to avoid overpressure and negative pressure inside the system depending on the direction imposed by the conditions of pressure between the inside and the outside of said recirculation circuit (6).

4. The device according to any one of the preceding claims, further comprising a regulating valve (2) at said supply port (3a), so as to limit set the pressure to the gas flow rate required by the application.

5. The device according to claim 4, wherein the regulating valve (2) on the supply port (3a) is a knob-type throttling element to adjust the air sucked from the environment and the inhaled fraction FiO2.

6. The device according to any one of the preceding claims, wherein said recirculation circuit (6) comprises a pressurization valve (11) configured to keep the pressure level in the ventilation chamber (C) above a predetermined threshold value.

7. The device according to claim 6, wherein the pressurization valve (11) comprises an inlet fitting (11a), placed in fluid connection with the expiratory duct (4b) of the patient interface element (4) and an outlet fitting (11b).

8. The device according to claim 6, wherein the pressurization valve (11) is arranged along the pipe (7), operationally upstream of said vent (9).

9. The device according to any one of the preceding claims, wherein said patient interface element (4) comprises a nasal mask, an oronasal mask, or a CPAP therapy hood.

10. The device according to any one of the preceding claims, wherein the flow generator (3) is defined, alternatively, by either a Venturi-effect-based system or a turbine.

11. The device according to any one of the preceding claims, further comprising at least one first flow meter (201, 301), associated with said flow generator (3) through at least one supply fitting (202).

12. The device according to claim 11, wherein said at least one first flow meter (201, 301) is a high-flow flow meter able to reach flow rates of at least 15 L/m.

13. The device according to claim 11 or 12, further comprising a second flow meter (204), adapted to adjust the titration value of the flow, comprising a flexible fitting (203) associable with said flow generator (3).

14. The device according to any one of the preceding claims 1 to 13, wherein said carbon dioxide absorber (8) comprises a zeolite element, a solid amine element or equivalent element, preferably a zeolite 13X element.

15. The device according to any one of claims 11 to 14, wherein said first flow meter (201, 301) is integrated with said flow generator (3) and generates an entrainment flow that determines the mixing of air and oxygen.

16. A recirculation assembly (100) for a non-invasive ventilation device, comprising:
- a pipe (107) developing between a first end (107a) and a second end (107b);
- a carbon dioxide absorber (108) arranged along said pipe (107);
- a vent (109) placed operationally upstream of the carbon dioxide absorber (108) and configured to avoid both negative pressure and overpressure inside the recirculation assembly;
- a pressurization valve (111) configured to keep the pressure level at the first end (107a) of the pipe (107) above a predetermined threshold value, arranged along the pipe (107) operationally upstream of said vent (109),
wherein said first (107a) and said second end (107b) of the pipe (107) are shaped to engage in an expiratory duct of a patient interface element and in a flow generator, respectively.

17. The recirculation assembly (100) according to claim 16, further comprising a flow generator (103) provided with at least one supply port (103a) which can be connected to a source (S) of a medical gas, with at least one dispensing port (103b) and with one recovery port (103c) connected to the second end (107b) of the pipe (107).

## Patentansprüche

1. Vorrichtung zur nicht-invasiven Beatmung und/oder Unterstützung des Patienten beim Atmen, umfassend:
- einen Flusserzeuger (3), der mit mindestens einer Zuführöffnung (3a), die mit einer Quelle (S) eines medizinischen Gases verbunden werden kann, und mit mindestens einer Ausgabeöffnung (3b) bereitgestellt ist;
- ein Patientenschnittstellenelement (4), das dazu geformt ist, eine Belüftungskammer (C) abzugrenzen, die mindestens die Nase des Patienten enthält, und das mit mindestens einem Inspirationskanal (4a), der mit dem Flusserzeuger (3) in Fluidverbindung steht, und einem Exspirationskanal (4b) bereitgestellt ist;
- einen Rezirkulationskreislauf (6), der sich zwischen dem Exspirationskanal (4b) des Patientenschnittstellenelements (4) und dem Flusserzeuger (3) entwickelt und dazu konfiguriert ist, mindestens einen Anteil des medizinischen Gases aus einer ausgeatmeten Mischung, die aus dem Exspirationskanal austritt, zurückzugewinnen,
wobei der Rezirkulationskreislauf (6) ein Rohr (7) umfasst, das sich zwischen einem ersten Ende (7a), das mit dem Exspirationskanal (4b) des Patientenschnittstellenelements (4) verbunden ist, und einem zweiten Ende (7b), das mit dem Flusserzeuger (3) verbunden ist, entwickelt; wobei der Rezirkulationskreislauf (6) ferner einen Kohlendioxidabsorber (8) umfasst, der entlang des Rohres (7) angeordnet ist, und wobei das Rohr (7) eine Entlüftung (9) umfasst, die betriebsmäßig stromaufwärts des Kohlendioxidabsorbers (8) platziert und dazu konfiguriert ist, sowohl Negativdruck als auch Überdruck innerhalb des Rezirkulationskreislaufs zu vermeiden.

2. Vorrichtung nach Anspruch 1, wobei der Flusserzeuger (3) mit einer Zurückgewinnungsöffnung (3c) versehen ist, die mit dem Rezirkulationskreislauf (6) verbunden ist, um den Anteil des medizinischen Gases aufzunehmen und ihn in Richtung der Ausgabeöffnung (3b) zu führen.

3. Vorrichtung nach Anspruch 1 oder 2, wobei die Entlüftung (9) dazu konfiguriert ist, Überdruck und Negativdruck innerhalb des Systems in Abhängigkeit von der Richtung, die durch die Druckbedingungen zwischen dem Inneren und dem Äußeren des Rezirkulationskreislaufs (6) vorgegeben wird, zu vermeiden.

4. Vorrichtung nach einem der vorhergehenden Ansprüche, ferner umfassend ein Regelventil (2) an der Zuführöffnung (3a), um eine Grenze des Druckes gemäß der Gasdurchflussrate, die durch die Anwendung angefordert wird, einzustellen.

5. Vorrichtung nach Anspruch 4, wobei das Regelventil (2) auf der Zuführöffnung (3a) ein Drosselelement vom Knauftyp ist, um die Luft, die aus der Umgebung angesogen wird, und die eingeatmete Fraktion Fi02 anzupassen.

6. Vorrichtung nach einem der vorhergehenden Ansprüche, wobei der Rezirkulationskreislauf (6) ein Druckbeaufschlagungsventil (11) umfasst, das dazu konfiguriert ist, den Druckpegel in der Belüftungskammer (C) über einem vorbestimmten Schwellenwert zu halten.

7. Vorrichtung nach Anspruch 6, wobei das Druckbeaufschlagungsventil (11) eine Einlassmuffe (11a), die in Fluidverbindung mit dem Exspirationskanal (4b) des Patientenschnittstellenelements (4) steht, und eine Auslassmuffe (11b) umfasst.

8. Vorrichtung nach Anspruch 6, wobei das Druckbeaufschlagungsventil (11) entlang des Rohres (7) betriebsmäßig stromaufwärts der Entlüftung (9) angeordnet ist.

9. Vorrichtung nach einem der vorhergehenden Ansprüche, wobei das Patientenschnittstellenelement (4) eine Nasenmaske, eine Mund-Nasenmaske oder eine CPAP-Therapiekappe umfasst.

10. Vorrichtung nach einem der vorhergehenden Ansprüche, wobei der Flusserzeuger (3) alternativ durch entweder ein Venturieffektbasiertes System oder eine Turbine definiert ist.

11. Vorrichtung nach einem der vorhergehenden Ansprüche, ferner umfassend mindestens einen ersten Durchflussmesser (201, 301), der durch mindestens eine Zuführmuffe (202) mit dem Flusserzeuger (3) assoziiert ist.

12. Vorrichtung nach Anspruch 11, wobei der mindestens eine erste Durchflussmesser (201, 301) ein Durchflussmesser für hohen Durchfluss ist, der Durchflussraten von mindestens 15 l/m zu erzielen vermag.

13. Vorrichtung nach Anspruch 11 oder 12, ferner umfassend einen zweiten Durchflussmesser (204), der dazu ausgelegt ist, den Titrationswert des Flusses anzupassen, und eine flexible Muffe (203) umfasst, die mit dem Flusserzeuger (3) assoziierbar ist.

14. Vorrichtung nach einem der vorhergehenden Ansprüche 1 bis 13, wobei der Kohlendioxidabsorber (8) ein Zeolithelement, ein Festaminelement oder gleichwertiges Element, vorzugsweise ein Zeolith-13X-Element umfasst.

15. Vorrichtung nach einem der Ansprüche 11 bis 14, wobei der erste Durchflussmesser (201, 301) mit dem Flusserzeuger (3) integriert ist und einen Mitreißfluss erzeugt, der das Mischen von Luft und Sauerstoff bestimmt.

16. Rezirkulationsanordnung (100) für eine nicht-invasive Beatmungsvorrichtung, umfassend:
- ein Rohr (107), das sich zwischen einem ersten Ende (107a) und einem zweiten Ende (107b) entwickelt;
- einen Kohlendioxidabsorber (108), der entlang des Rohres (107) angeordnet ist;
- eine Entlüftung (109), die betriebsmäßig stromaufwärts des Kohlendioxidabsorbers (108) platziert und dazu konfiguriert ist, sowohl Negativdruck als auch Überdruck innerhalb der Rezirkulationsanordnung zu vermeiden;
- ein Druckbeaufschlagungsventil (111), das dazu konfiguriert ist, den Druckpegel an dem ersten Ende (107a) des Rohres (107) über einem vorbestimmten Schwellenwert zu halten, und entlang des Rohres (107) betriebsmäßig stromaufwärts der Entlüftung (109) angeordnet ist,
wobei das erste (107a) und das zweite Ende (107b) des Rohres (107) dazu geformt sind, in einen Exspirationskanal eines Patientenschnittstellenelements beziehungsweise in einen Flusserzeuger einzugreifen.

17. Rezirkulationsanordnung (100) nach Anspruch 16, ferner umfassend einen Flusserzeuger (103), der mit mindestens einer Zuführöffnung (103a), die mit einer Quelle (S) eines medizinischen Gases verbunden werden kann, mit mindestens einer Ausgabeöffnung (103b) und mit einer Zurückgewinnungsöffnung (103c), die mit dem zweiten Ende (107b) des Rohres (107) verbunden ist, bereitgestellt ist.

## Revendications

1. Dispositif pour la ventilation non invasive et/ou pour faciliter la respiration du patient, comprenant:
- un générateur de flux (3) muni d'au moins un orifice d'alimentation (3a) qui peut être relié à une source (S) d'un gaz médical et d'au moins un orifice de distribution (3b);
- un élément d'interface patient (4) conformé pour délimiter une chambre de ventilation (C) qui contient au moins le nez du patient et muni d'au moins un conduit inspiratoire (4a), en liaison fluidique avec ledit générateur de flux (3), et d'un conduit expiratoire (4b);
- un circuit de recirculation (6) se développant entre ledit conduit expiratoire (4b) de l'élément d'interface patient (4) et le générateur de flux (3) et configuré pour récupérer au moins une partie dudit gaz médical à partir d'un mélange exhalé sortant dudit conduit expiratoire,
dans lequel ledit circuit de recirculation (6) comprend un tube (7) se développant entre une première extrémité (7a), reliée au conduit expiratoire (4b) de l'élément d'interface patient (4), et une deuxième extrémité (7b), reliée au générateur de flux (3); ledit circuit de recirculation (6) comprenant en outre un absorbeur de dioxyde de carbone (8) disposé le long dudit tube (7), et
dans lequel le tube (7) comprend un évent (9) placé fonctionnellement en amont de l'absorbeur de dioxyde de carbone (8) et configuré pour éviter une pression négative et une surpression à l'intérieur du circuit de recirculation.

2. Dispositif selon la revendication 1, dans lequel ledit générateur de flux (3) est équipé d'un orifice de récupération (3c) relié au circuit de recirculation (6) pour recevoir ladite partie du gaz médical et la diriger vers l'orifice de distribution (3b).

3. Dispositif selon la revendication 1 ou 2, dans lequel ledit évent (9) est configuré pour éviter une surpression et une pression négative à l'intérieur du système en fonction du sens imposé par les conditions de pression entre l'intérieur et l'extérieur dudit circuit de recirculation (6).

4. Le dispositif selon l'une quelconque des revendications précédentes, comprenant en outre une vanne de régulation (2) au niveau de l'orifice d'alimentation (3a), de manière à limiter la pression au débit de gaz requis par l'application.

5. Dispositif selon la revendication 4, dans lequel la vanne de régulation (2) sur l'orifice d'alimentation (3a) est un élément d'étranglement de type bouton pour ajuster l'air aspiré depuis l'environnement et la fraction inhalée FiO2.

6. Dispositif selon l'une quelconque des revendications précédentes, dans lequel ledit circuit de recirculation (6) comprend une vanne de mise en pression (11) configurée pour maintenir le niveau de pression dans la chambre de ventilation (C) au-dessus d'une valeur seuil prédéterminée.

7. Dispositif selon la revendication 6, dans lequel la vanne de mise en pression (11) comprend un raccord d'entrée (11a), placé en liaison fluidique avec le conduit expiratoire (4b) de l'élément d'interface patient (4) et un raccord de sortie (11b).

8. Dispositif selon la revendication 6, dans lequel la vanne de mise en pression (11) est disposée le long du tube (7), fonctionnellement en amont dudit évent (9).

9. Dispositif selon l'une quelconque des revendications précédentes, dans lequel ledit élément d'interface patient (4) comprend un masque nasal, un masque oronasal ou une cagoule de thérapie CPAP.

10. Dispositif selon l'une quelconque des revendications précédentes, dans lequel le générateur de flux (3) est défini, alternativement, par un système basé sur l'effet Venturi ou une turbine.

11. Dispositif selon l'une quelconque des revendications précédentes, comprenant en outre au moins un premier débitmètre (201, 301), associé audit générateur de flux (3) par l'intermédiaire d'moins un raccord d'alimentation (202).

12. Dispositif selon la revendication 11, dans lequel ledit au moins un premier débitmètre (201, 301) est un débitmètre à haut flux capable d'atteindre des débits d'au moins 15 L/m.

13. Dispositif selon la revendication 11 ou 12, comprenant en outre un deuxième débitmètre (204), adapté pour ajuster la valeur de titrage du flux, comprenant un raccord flexible (203) pouvant être associé audit générateur de flux (3).

14. Dispositif selon l'une quelconque des revendications 1 à 13 précédentes, dans lequel ledit absorbeur de dioxyde de carbone (8) comprend un élément zéolite, un élément amine solide ou un élément équivalent, de préférence un élément zéolithe 13X.

15. Dispositif selon l'une quelconque des revendications 11 à 14, dans lequel ledit premier débitmètre (201, 301) est intégré audit générateur de flux (3) et génère un flux d'entraînement qui détermine le mélange d'air et d'oxygène.

16. Ensemble de recirculation (100) pour un dispositif de ventilation non invasive, comprenant:
- un tube (107) se développant entre une première extrémité (107a) et une deuxième extrémité (107b);
- un absorbeur de dioxyde de carbone (108) disposé le long dudit tube (107);
- un évent (109) placé fonctionnellement en amont de l'absorbeur de dioxyde de carbone (108) et configuré pour éviter à la fois une pression négative et une surpression à l'intérieur de l'ensemble de recirculation;
- une vanne de mise en pression (111) configurée pour maintenir le niveau de pression au niveau de la première extrémité (107a) du tube (107) au-dessus d'une valeur seuil prédéterminée, disposée le long du tube (107) fonctionnellement en amont dudit évent (109),
dans lequel ladite première (107a) et ladite deuxième extrémité (107b) du tube (107) sont conformées pour s'engager dans un conduit expiratoire d'un élément d'interface patient et dans un générateur de flux, respectivement.

17. Ensemble de recirculation (100) selon la revendication 16, comprenant en outre un générateur de flux (103) muni d'au moins un orifice d'alimentation (103a) qui peut être relié à une source (S) d'un gaz médical, d'au moins un orifice de distribution (103b) et d'un orifice de récupération (103c) relié à la deuxième extrémité (107b) du tube (107).
